# EUROPEAN PATENT APPLICATION

(11) **EP 4 276 766 A2**
(43) Date of publication of application: **15.11.2023**
(21) Application number: 23173208.2
(22) Date of filing: 12.05.2023
(51) Int. Cl.: G06T 19/20, G06T 15/04

(54) **METHOD, SERVER AND COMPUTER PROGRAM FOR PERFORMING FACE TRANSFORMATION SIMULATION**

(30) Priority: 13.05.2022 KR 20220058974
(71) Applicant: 3D ONS, INC., Seoul 06023 (KR)
(72) Inventor: OH, Seung Young, 06023 Seoul (KR)
(74) Representative: Walaski, Jan Filip

(57) **Abstract**

Provided is a face transformation simulation method. The method is performed by one or more processors of a computing device, and includes obtaining three-dimensional (3D) volume data and 3D facial data, generating matching data by matching the 3D volume data and the 3D facial data, generating a user interface including the matching data and providing the user interface to a user terminal, and performing transformation on the matching data on the basis of a response to the user interface, which is received from the user terminal.

## Description

### CROSS-REFERENCE TO RELATED APPLICATION

This application claims priority to Korean Patent Application No. 2022-0058974, filed on May 13, 2022.

### BACKGROUND

### 1. Field of the Invention

Various embodiments of the present disclosure relate to a face transformation simulation, and more particularly, to a method, a server, and a computer program for simulating a change in soft tissue corresponding to a change in hard tissue in a procedure or surgery.

### 2. Discussion of Related Art

In recent years, as social perceptions of plastic surgery and correction have changed, plastic and orthognathic surgery has been performed. Plastic and orthognathic surgery provides users with functional satisfaction as well as aesthetic satisfaction.

In general, in the case of plastic and orthognathic surgery, medical consultation is required to show a change in the face after the surgery to a person who will undergo the surgery and thus a surgery simulation is performed to predict a result after the surgery. When the surgery simulation is performed, an aesthetic change before and after the plastic and orthognathic surgery can be shown to a person (e.g., a doctor) who will perform the surgery or a person (e.g., a patient) who will undergo the surgery. To this end, in medical facilities such as a dental clinic or plastic surgery clinic, equipment for simulating clinical images of changes after treatment are used. A clinical image is an optical image (a photograph, etc.) of a target treatment area of a person who undergoes treatment, and changes before and after the treatment may be displayed on a corresponding device through a simulation. Korean Laid-open Patent Application No. 10-2018-0026029 discloses a method of performing a virtual plastic and orthognathic surgery simulation.

However, in existing clinical image simulation methods, a method of simply reflecting an operator's image manipulation result in a corresponding optical image is used. This method is only a technique for processing an image according to an operator's image manipulation and displaying a resultant image, and therefore, a simulation result may vary according to the operator's individual capability, the accuracy of an inspection may be low, and the method may be inconvenient to be used for a simulation.

### SUMMARY OF THE INVENTION

To address the aforementioned background technology, the present disclosure is directed to providing a method of simulating a change in soft tissue corresponding to a change in hard tissue in a procedure or surgery.

Aspects of the present disclosure are not limited thereto and other aspects that are not described here will be clearly understood by those of ordinary skill in the art from the following description.

An aspect of the present disclosure provides a face transformation simulation method.

The method is performed by one or more processors of a computing device, and includes obtaining three-dimensional (3D) volume data and 3D facial data, generating matching data by matching the 3D volume data and the 3D facial data, generating a user interface including the matching data and providing the user interface to a user terminal, and performing transformation on the matching data on the basis of a response to the user interface, which is received from the user terminal.

In an alternative embodiment, the 3D volume data may be volume data related to the head and neck of a person who undergoes surgery and include one or more landmarks, and the 3D facial data may be image data related to the face of the person who undergoes the surgery and include polygon data and texture data.

In an alternative embodiment, the generating of the matching data may include identifying landmarks for matching in the 3D volume data and the 3D facial data, and generating the matching data on the basis of the identified landmarks for matching.

In an alternative embodiment, the user interface may include a simulation selection screen for receiving a selection input related to an orthodontic simulation or a plastic and orthognathic surgery simulation from a user, a matching data display screen displaying the matching data, and a hard tissue change information input screen for receiving a hard tissue adjustment input in relation to a change in hard tissue from the user.

In an alternative embodiment, the performing of the transformation on the matching data may include changing the hard tissue on the basis of the hard tissue adjustment input, and changing soft tissue corresponding to the hard tissue using a weighted array when the hard tissue is changed, and the weighted array may calculate a movement coefficient of the soft tissue according to the change in the hard tissue.

In an alternative embodiment, the changing of the hard tissue may be performed through a plurality of surgical operations, and the plurality of surgical operations may include a first surgical operation of performing an x-axis movement, a second surgical operation of performing a z-axis rotation with respect to an arbitrary reference point after applying the first surgery operation with respect to one or more landmarks, a third surgical operation of performing a y-axis rotation with respect to the arbitrary reference point after applying the second surgery operation with respect to the one or more landmarks, a fourth surgical operation of performing a z-axis movement, a fifth surgical operation of performing a x-axis rotation with respect to the arbitrary reference point after applying the fourth surgery operation with respect to the one or more landmarks, and a sixth surgical operation of performing a y-axis movement.

In an alternative embodiment, the weighted array may include a mesh including a plurality of vertices and a plurality of edges connecting the plurality of vertices, and the plurality of vertices may be matched to setting values according to a positional relationship with one or more landmarks.

In an alternative embodiment, when a force is applied to the plurality of vertices according to movement of the hard tissue, the weighted array may move the plurality of vertices on the basis of the applied force and the setting values matched to the plurality of vertices, and the plurality of vertices may be assigned different weights according to depths of connection between adjacent vertices.

Another aspect of the present disclosure provides a server for performing a face transformation simulation method. The server may include a memory storing one or more instructions, and a processor configured to execute the one or more instructions stored in the memory, and the processor executes the one or more instructions to perform the method described above.

Another aspect of the present disclosure provides a computer program stored in a computer-readable recording medium. The computer program may perform a face transformation simulation method in combination with a computer which is hardware.

Other aspects of the present disclosure will be apparent from the detailed description and drawings.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a diagram schematically illustrating a system for performing a face transformation simulation method according to an embodiment of the present disclosure;
FIG. 2 illustrates a hardware configuration of a server for performing a face transformation simulation method according to an embodiment of the present disclosure;
FIG. 3 is a diagram illustrating an overall process of a face transformation simulation according to an embodiment of the present disclosure;
FIG. 4 is a flowchart of a face transformation simulation method according to an embodiment of the present disclosure;
FIG. 5 is a diagram illustrating a user interface according to an embodiment of the present disclosure;
FIG. 6 is a diagram illustrating a user interface according to another embodiment of the present disclosure;
FIG. 7 is a diagram illustrating a three-dimensional (3D) axis related to a face according to an embodiment of the present disclosure;
FIG. 8 is a diagram illustrating a plurality of surgical operations according to an embodiment of the present disclosure;
FIG. 9 is a diagram illustrating a mesh included in a weighted array according to an embodiment of the present disclosure;
FIG. 10A is a diagram for describing a weighted array according to an embodiment of the present disclosure;
FIG. 10B is a diagram for describing a weighted array according to an embodiment of the present disclosure;FIG. 11 is a diagram illustrating a change in a weighted array according to an embodiment of the present disclosure;
FIG. 12A is a diagram illustrating, in detail, a change in soft tissue according to a change in hard tissue according to an embodiment of the present disclosure; and
FIG. 12B is a diagram illustrating, in detail, a change in soft tissue according to a change in hard tissue according to an embodiment of the present disclosure.

### DETAILED DESCRIPTION OF EXEMPLARY EMBODIMENTS

Hereinafter, various embodiments will be described with reference to the accompanying drawings. In the present specification, various examples are provided to help an understanding of the present disclosure. However, it will be clear that these embodiments can be implemented without such a detailed description.

The term "component," "module," "system," or the like, when used herein, refers to a computer-related entity, hardware, firmware, software, a combination of software and hardware, or execution of software. For example, a component may be understood as, but is not limited to, a procedure performed by a processor, a processor, an object, an execution thread, a program, and/or a computer. For example, both an application executed by a computing device and the computing device may be components. One or more components may reside in a processor and/or an execution thread. One component may be localized in one computer. One component may be distributed between two or more computers. These components may be run from various computer-readable media storing various data structures therein. Components may communicate with each other through local and/or remote processing, for example, according to a signal containing one or more data packets (e.g., data transmitted from a system through a network such as the Internet according to data and/or a signal from one component interacting with another component in a local or distributed system).

In addition, the term "or" is intended to mean an inclusive "or" rather than an exclusive "or". That is, "X uses A or B" is intended to mean one of natural inclusive substitutions unless otherwise specified or contextually clear. That is, "X uses A or B" should be understood to mean that X uses A, X uses B, or X uses both A and B. The term "and/or" when used herein should be understood to refer to and include all possible combinations of one or more of relevant items listed herein.

In addition, the terms "comprise" and/or "comprising" should be understood to mean the presence of a corresponding feature and/or component. However, it will be understood that the terms "comprise" and/or "comprising" do not preclude the presence or addition of one or more other features, components, and/or groups thereof. Each singular form described in the detailed description and claims should be understood to generally mean "one or more" unless otherwise specified or the context clearly indicates a singular form.

It will be understood by those of ordinary skill in the art that various examples of logical blocks, configurations, modules, circuits, means, logic, and operations of an algorithm additionally described below in relation to embodiments set forth herein can be implemented by electronic hardware, computer software, or a combination thereof. To clearly indicate the interchangeability of hardware and software, various examples of components, blocks, configurations, means, logic, modules, circuits, and operations have generally been described above in terms of functionalities thereof. Whether to implement such functionality by hardware or software depends on specific applications and design limitations imposed on an overall system. It will be obvious that skilled technicians can implement functionalities in various ways for each specific application. However, it should not be understood that decisions of such implementation do depart from the scope of the present disclosure.

A description of embodiments set forth herein is provided to help those of ordinary skill in the art use or implement the present disclosure. It will be apparent to those of ordinary skill in the art that various modifications can be made in these embodiments. General principles defined herein may apply to other embodiments without departing from the scope of the present disclosure. Therefore, the present disclosure is not limited to embodiments set forth herein. The present disclosure should be interpreted within a broadest range consistent with principles and novel features described herein.

As used herein, the term "computer" should be understood to mean various types of hardware devices, including at least one processor, and may be understood to include a software component operating in a corresponding hardware device according to an embodiment. For example, a computer may be understood to include, but is not limited to, a smart phone, a tablet PC, a desktop computer, a laptop computer, and a user client and an application running on each device.

Hereinafter, embodiments of the present disclosure will be described in detail with reference to the accompanying drawings.

Operations described herein will be described as being performed by a computer, but a subject of each of the operations is not limited thereto and at least some of the operations may be performed by different devices according to an embodiment.

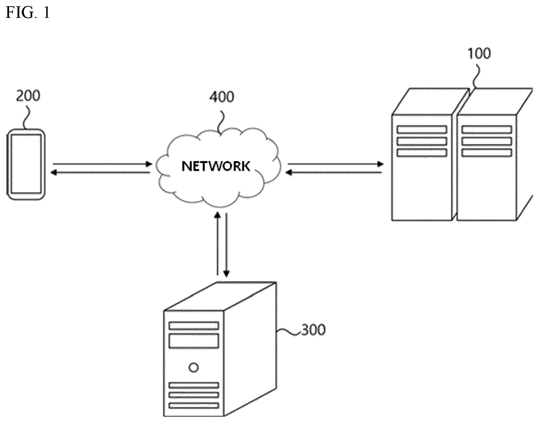

FIG. 1 is a diagram schematically illustrating a system for performing a face transformation simulation method according to an embodiment of the present disclosure.

As illustrated in FIG. 1, a system according to embodiments of the present disclosure may include a server 100, a user terminal 200, an external server 300, and a network 400. The components shown in FIG. 1 are only examples and thus other components may be added or some of the components shown in FIG. 1 may be omitted. The server 100, the external server 300, and the user terminal 200 according to embodiments of the present disclosure may mutually transmit or receive data for the system according to an embodiment of the present disclosure through the network 400.

The network 400 according to embodiments of the present disclosure may use any of various types of wired communication systems such as a public switched telephone network (PSTN), x digital subscriber line (xDSL), rate adaptive DSL (RADSL), multi-rate DSL (MDSL), very high Speed DSL (VDSL), universal asymmetric DSL (UADSL), high bit rate DSL (HDSL), and a local area network (LAN).

In addition, the network 400 described herein may use any of various types of wireless communication systems such as code division multi access (CDMA), time division multi access (TDMA), frequency division multi access (FDMA), orthogonal frequency division multi access (OFDMA), single carrier-FDMA (SC-FDMA), and other systems.

The network 400 according to embodiments of the present disclosure may be configured regardless of the type of communication mode, e.g., wired communication or wireless communication, and configured of any of various types of communication networks such as a personal area network (PAN) and a wide area network (WAN). In addition, the network 400 may be the well-known World Wide Web (WWW) and may use wireless transmission technology, e.g., Infrared Data Association (IrDA) or Bluetooth, used for short-range communication. Technologies described herein may be used not only in the networks described above but also in other networks.

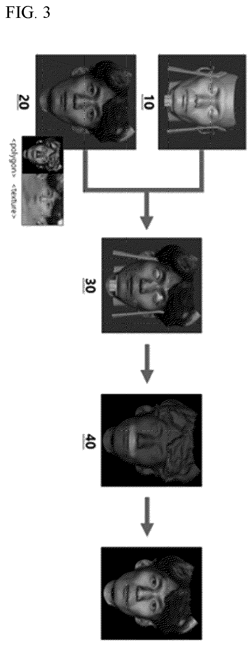

According to an embodiment of the present disclosure, the server 100 that performs the face transformation simulation method (hereinafter referred to as the "server 100") may generate matching data 30 and perform transformation on the matching data 30 to provide face transformation simulation information. In an embodiment, referring to FIG. 3, the matching data 30 is three-dimensional (3D) data related to the face of a person who will undergo surgery, and may be generated by matching 3D volume data 10 and 3D facial data 20 related to the person. The face transformation simulation information is related to virtual surgery related to plastic and orthognathic surgery, and may include information about a change in soft tissue according to a change in hard tissue as a result of a surgery simulation. Specifically, the server 100 may generate the matching data 30 by matching the 3D volume data 10 and the 3D facial data 20, and provide a user interface including the matching data 30. Here, the user interface may be intended to obtain various types of information from a user of the user terminal 200 for performing a surgery and face transformation simulation or to provide data generated as a result of performing the surgery and face transformation simulation. The server 100 may perform a simulation related to surgery and face transformation based on a response to the user interface, which is received from the user terminal 200.

In general, a face transformation simulation provided in a dental clinic or plastic surgery clinic simply uses a method of simply reflecting an operator's image manipulation for a corresponding optical image. This method is only a technique for processing an image according to an operator's image manipulation and displaying a resultant image, and therefore, a simulation result may vary according to the operator's individual capability, the accuracy of an inspection may be low, and the method may be inconvenient to be used for a simulation.

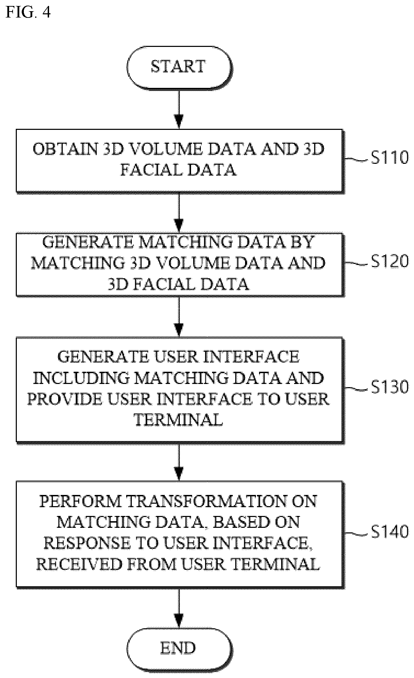

The server 100 of the present disclosure may perform a virtual surgery simulation (e.g., a surgery simulation) to change hard tissue with respect to one or more landmarks in matching data corresponding to a person who will undergo surgery instead of directly correcting soft tissue, and perform a simulation (i.e., a face transformation simulation) to change the soft tissue according to the change in the hard tissue as a result of the virtual surgery simulation. In this case, as shown in FIG. 3, the server 100 may provide a simulation in which a weighted array 40 is generated for a surgical site on the basis of one or more landmarks and soft tissue (e.g., skin) is changed according to movement of hard tissue (e.g., bone or dentition), using the weighted array 40. Accordingly, a user (e.g., a person who will perform surgery) may obtain information about a change in soft tissue only on the basis of a change in hard tissue reflected as a result of performing a simulation related to the surgery without direct correction of the soft tissue, thereby improving convenience. The face transformation simulation method performed by the server 100 will be described in detail with reference to FIG. 4 below.

In an embodiment, although FIG. 1 illustrates only one server 100, it will be apparent to those of ordinary skill in the art that more than one server falls within the scope of the present disclosure and the server 100 may include additional components. That is, the server 100 may include a plurality of computing devices. In other words, a set of nodes may constitute the server 100.

According to an embodiment of the present disclosure, the server 100 may be a server that provides a cloud computing service. More specifically, the server 100 may be a server that provides a cloud computing service for processing information using a computer connected to the Internet rather than a user's computer, i.e., a type of Internet-based computing. The cloud computing service may be a service for storing data on the Internet and allowing a user to use desired data or a desired program regardless of time and place by connecting to the Internet without storing the desired data or program on the user's computer, and the data stored on the Internet can be easily shared and delivered through simple manipulation and clicking. In addition, the cloud computing service may be a service for allowing a desired task to be performed using functions of an application program provided on the web without additionally installing a program and allowing several persons to perform a task at the same time while sharing a document, as well as simply storing data on a server on the Internet. The cloud computing service may be implemented in the form of at least one of infrastructure as a service (IaaS), platform as a service (PaaS), software as a service (SaaS), a virtual machine-based cloud server, or a container-based cloud server. That is, the server 100 of the present disclosure may be implemented in the form of at least one of the above-described cloud computing services. The above-described cloud computing services are only examples and may include any platform for constructing a cloud computing environment of the present disclosure.

The user terminal 200 according to the embodiment of the present disclosure may be understood as any type of node(s) in a system having a mechanism for communication with the server 100. The user terminal 200 is a terminal capable of receiving information about a face transformation simulation through the exchange of information with the server 100 and may be understood as a user's terminal. For example, the user terminal 200 may be a terminal related to a user (a person who will perform or undergo surgery) for obtaining information about a degree of change in the face when plastic and orthognathic surgery is to be performed.

In an embodiment, the user terminal 200 may be connected to the server 100 through the network 400, provide the server 100 with a plurality of pieces of data (e.g., 3D volume data and 3D facial data), and may be supplied with results of a surgery simulation and a face transformation simulation related to matching data in response to the provided pieces of data.

The user terminal 200 may be understood as any type of entity(s) in a system having a mechanism for communication with the server 100. Examples of the user terminal 200 may include a personal computer (PC), a notebook computer, a mobile terminal, a smart phone, a tablet PC, a wearable device, etc., and include various types of devices capable of accessing a wired/wireless network. Examples of the user terminal 200 may include a server implemented by at least one of an agent, an application programming interface (API) or a plug-in. In addition, examples of the user terminal 200 may include an application source and/or a client application.

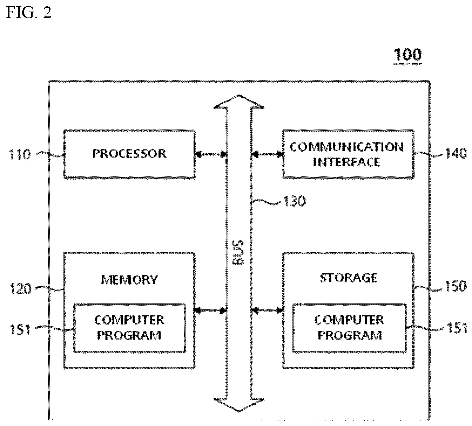

In an embodiment, the external server 300 may be connected to the server 100 through the network 400 and supplied with resulting data obtained as the server 100 provides various types of information/data necessary to perform the face transformation simulation method or performs the face transformation simulation method, and store and manage the resulting data. For example, the external server 300 may be a storage server provided separately outside the server 100 but is not limited thereto. A hardware configuration of the server 100 that performs a face transformation simulation method will be described with reference to FIG. 2 below.

FIG. 2 illustrates a hardware configuration of a server for performing a face transformation simulation method according to an embodiment of the present disclosure.

Referring to FIG. 2, a server 100 that performs the face transformation simulation method according to the embodiment of the present disclosure may include one or more processors 110, a memory 120 configured to load therein a computer program 151 to be executed by the one or more processors 110, a bus 130, a communication interface 140, and a storage 150 storing the computer program 151. Here, FIG. 2 illustrates only components related to the embodiment of the present disclosure. Therefore, it will be apparent to those of ordinary skill in the art that other general-purpose components may be further provided, as well as the components illustrated in FIG. 2.

According to an embodiment of the present disclosure, the processor 110 may generally control the overall operation of the server 100. The processor 110 may process signals, data, information, and the like that are input or output through the components described above or may execute an application program stored in the memory 120 to provide appropriate information or functions to a user or a user terminal or process the information or functions.

The processor 110 may perform an operation on at least one application or program for performing methods according to embodiments of the present disclosure, and the server 100 may include one or more processors.

According to an embodiment of the present disclosure, the processor 110 may include one or more cores, and include a processor for analyzing data and performing deep learning, e.g., a central processing unit (CPU), a general-purpose graphics processing unit (GPGPU) or a tensor processing unit (TPU).

The processor 110 may read a computer program stored in the memory 120 and perform the face transformation simulation method according to the embodiment of the present disclosure.

In various embodiments, the processor 110 may further include a random access memory (RAM) (not shown) and a read-only memory (ROM) (not shown) for temporarily and/or permanently storing signals (or data) to be processed in the processor 110. The processor 110 may be in the form of a system-on-chip (SoC) including at least one of a graphics processor, a RAM, or a ROM.

The memory 120 stores various types of data, instructions, and/or information. The memory 120 may load the computer program 151 from the storage 150 to perform methods/operations according to various embodiments of the present disclosure. When the computer program 151 is loaded in the memory 120, the processor 110 may execute one or more instructions constituting the computer program 151 to perform the methods/operations. The memory 120 may be embodied as a volatile memory such as a RAM, but the technical scope of the present disclosure is not limited thereto.

The bus 130 provides a communication function between the components of the server 100. The bus 130 may be embodied as any of various types of buses such as an address bus, a data bus, and a control bus.

The communication interface 140 supports wired/wireless Internet communication of the server 100. The communication interface 140 may support various communication methods other than Internet communication. To this end, the communication interface 140 may include a communication module well known in the technical field of the present disclosure. In some embodiments, the communication interface 140 may be omitted.

The storage 150 may store the computer program 151 non-temporarily. When a process of performing a face transformation simulation is performed through the server 100, the storage 150 may store various types of information required to provide the process of performing the face transformation simulation.

The storage 150 may include a non-volatile memory, such as a ROM, an erasable programmable ROM (EPROM), an electrically erasable programmable ROM (EEPROM) or a flash memory, a hard disk, a detachable disk, or any type of computer-readable recording medium well known in the technical field to which the present disclosure pertains.

The computer program 151 may include one or more instructions causing the processor 110 to perform methods/operations according to various embodiments of the present disclosure when the computer program 151 is loaded in the memory 120. That is, the processor 110 may execute the one or more instructions to perform the method/operations according to various embodiments of the present disclosure.

In an embodiment, the computer program 151 may include one or more instructions to perform a face transformation simulation method including obtaining 3D volume data and 3D facial data, generating matching data by matching the 3D volume data and the 3D facial data, generating a user interface including the matching data and providing the user interface to a user terminal, and performing transformation on the matching data on the basis of a response to the user interface, received from the user terminal.

The operations of the method or an algorithm described above in connection with embodiments of the present disclosure may be implemented directly by hardware, a software module executed by hardware, or a combination thereof. The software module may reside in a RAM, a ROM, an EPROM, an EEPROM, a flash memory, a hard disk, a removable disk, a CD-ROM, or any type of computer-readable recording medium well-known in the technical field to which the present disclosure pertains.

Components of the present disclosure may be embodied in the form of a program (or an application) and stored in a medium to be executed in combination with a computer which is hardware. The components of the present disclosure may be implemented by software programming or software elements, and similarly, embodiments may be implemented in a programming or scripting language such as C, C++, Java, or an assembler, including data structures, processes, routines, or various algorithms which are combinations of different programming constructs. Functional aspects may be embodied as an algorithm executable by one or more processors. A face transformation simulation method performed by the server 100 will be described in detail with reference to FIGS. 4 to 12 below.

FIG. 4 is a flowchart of a face transformation simulation method according to an embodiment of the present disclosure. An order of the operations illustrated in FIG. 4 may be changed as necessary and at least one operation may be omitted or added. That is, the operations to be described below are only examples of the present disclosure and the scope of the present disclosure is not limited thereto.

According to an embodiment of the present disclosure, the server 100 may obtain 3D volume data and 3D facial data (S110). As shown in FIG. 3, the 3D volume data 10 may be volume data related to a user's head and neck and include one or more landmarks. The one or more landmarks are related to anatomical points on the head of neck of a person who will undergo surgery and may be related to, for example, the forehead, the nose, the orbitale, a specific tooth, or the like. For example, one or more landmarks according to the present disclosure may be identified through an image recognition model or based on a user input related to a 3D image. Here, the image recognition model may be a neural network model trained to recognize a specific area (e.g., a landmark) of an image. In an embodiment, the server 100 of the present disclosure may provide a user interface for setting a landmark (e.g., a landmark recognition user interface) to the user terminal 200, and the user of the user terminal 200 may set a landmark in relation to a specific area of an image using the user interface. The detailed description of the method of setting a landmark is only an example, and the present disclosure is not limited thereto.

The 3D facial data 20 is image data related to the face of a person who will undergo surgery and may include polygon data and texture data as shown in FIG. 3. In an embodiment, the 3D facial data 20 may be obtained by a 3D scan associated with the face of the person who will undergo the surgery.

In an embodiment, the server 100 may receive the 3D volume data 10 and the 3D facial data 20 through the communication interface 140. In an embodiment of the present disclosure, the receiving of the 3D volume data 10 and the 3D facial data 20 may be receiving or loading the 3D volume data 10 and the 3D facial data 20 stored in the memory 120. The receiving of the 3D volume data 10 and the 3D facial data 20 may be receiving data from an additional processing module included in a different computing device or the same computing device or loading data in a different storage medium through a wired/wireless communication device.

According to an embodiment of the present disclosure, the server 100 may generate matching data by matching the 3D volume data and the 3D facial data (S120). Specifically, the server 100 may identify a landmark for matching in each of the 3D volume data 10 and the 3D facial data 20. The landmark for matching is information serving as a reference point for matching pieces of data and may be generated through a user input. The server 100 may set landmarks for matching in the 3D volume data 10 and the 3D facial data 20 in advance on the basis of a landmark-for-matching-related user input from the user terminal 200, and identify the set matching landmarks for matching between the 3D volume data 10 and the 3D facial data 20. The landmarks for matching may be formed by, for example, drawing lines to sequentially connect the eyes, the nose, and the mouth in the 3D volume data 10 and the 3D facial data 20. The server 100 may generate matching data on the basis of the identified landmarks for matching. That is, the server 100 may identify the landmarks for matching, which are set in the 3D volume data 10 and the 3D facial data 20, and generate the matching data by matching between the 3D volume data 10 and the 3D facial data 20 on the basis of the landmarks (e.g., so that the landmarks for matching may overlap each other).

According to an embodiment of the present disclosure, the server 100 may generate a user interface including the matching data and provide the user interface to a user terminal (S130).

According to an embodiment, a user interface 500 may provide a surgery simulation related to a change in hard tissue and a face transformation simulation related to a change in soft tissue according to the change in the hard tissue. The user interface 500 may receive a user input for performing the surgery simulation and perform transformation on the matching data on the basis of the user input. Here, the transformation on the matching data may be understood as transformation on hard tissue and soft tissue.

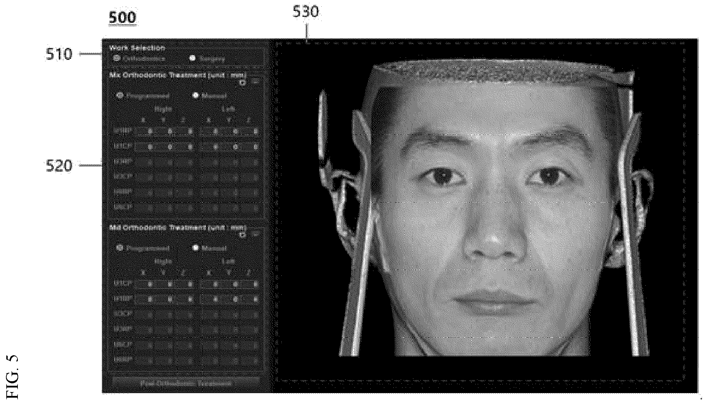

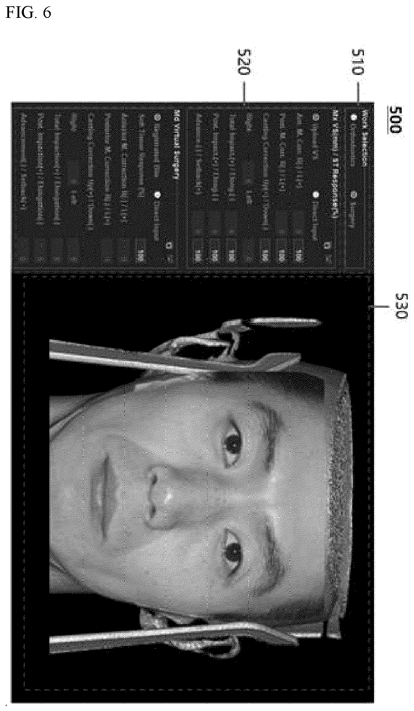

Specifically, as shown in FIGS. 5 and 6, the user interface 500 may include a simulation selection screen 510 for receiving a selection input related to an orthognathic simulation or a plastic and orthognathic surgery simulation from a user. In an embodiment, an orthodontic simulation may be performed when a selection input related to orthodontics is received from a user, and a plastic and orthognathic surgery simulation may be performed when a selection input related to plastic and orthognathic surgery is received from the user. The orthodontic simulation may include a simulation related to correction. According to an embodiment, the orthodontic simulation may be a simulation in which teeth corresponding to the upper jaw and teeth corresponding to the lower jaw are individually segmented and relocated according to 3D movement of each of the teeth. As shown in FIG. 5, when orthodontic correction is selected on the simulation selection screen 510, an input window for receiving a setting value for relocating each of the teeth on the upper jaw and the teeth on the lower jaw may be displayed on a hard tissue change information input screen 520.

In an embodiment, a plastic and orthognathic surgery simulation supported by the user interface 500 may include a plastic and orthognathic surgery simulation related to the upper and lower jaws (e.g., maxilla. mandible, chin, proximal segment, etc.), a plastic and orthognathic surgery simulation related to zygoma, and a plastic and orthognathic surgery simulation related to square jaw (gonial angle). The plastic and orthognathic surgery simulations may be performed on the basis of a user's selection input for at least one of various surgical areas. For example, an upper jaw area may be determined as a simulation target according to a user's selection. As shown in FIG. 6, when plastic surgery is selected on the simulation selection screen 510, an input window for receiving a setting value for adjusting the position of a surgical site (e.g., the upper jaw) with respect to one or more landmarks (e.g., orbitale, PNS, U1MP, U6MP, etc.) may be displayed on the hard tissue change information input screen 520.

According to an embodiment, data to be displayed on the hard tissue change information input screen 520 may vary depending on an item selected on the simulation selection screen 510 by a user. When a selection input related to orthodontics is received from a user through the simulation selection screen 510, a first input window for receiving a setting value for orthodontics may be displayed on the hard tissue change information input screen 520 as shown in FIG. 5, and when a selection input related to surgery is received from the user through the simulation selection screen 510, a second input window for receiving a setting value for plastic surgery may be displayed on the hard tissue change information input screen 520 as shown in FIG. 6.

According to an embodiment, the user interface 500 may include the hard tissue change information input screen 520 for receiving a hard tissue adjustment input in relation to a change in hard tissue from a user. The hard tissue change information input screen 520 may include an input window for receiving a setting value for adjusting the position of the teeth, the upper and lower jaws or the like on the basis of one or more landmarks. As shown in FIG. 5, the hard tissue change information input screen 520 may include an input window for receiving a setting value for individually and three-dimensionally moving teeth corresponding to the upper jaw and teeth corresponding to the lower jaw. In addition, the hard tissue change information input screen 520 may include an input window for receiving a setting value for adjusting the upper or lower jaw as shown in FIG. 6.

The user interface 500 may further include a matching data display screen 530 for displaying matching data. The matching data display screen 530 may display the matching data 30. The matching data display screen 530 may display the matching data 30 that changes as a simulation is performed through an input related to surgery or correction. That is, the matching data display screen 530 may display the correction or surgery, which is performed on the basis of a user input related to the simulation selection screen 510 and the hard tissue change information input screen 520, through simulation based on the matching data 30.

According to an embodiment of the present disclosure, the server 100 may perform transformation on the matching data on the basis of a response to the user interface, received from the user terminal (S140).

The server 100 may change hard tissue on the basis of a hard tissue adjustment input. Specifically, the server 100 may perform a surgery simulation on the basis of the hard tissue adjustment input from the user in relation to the matching data display screen 530, thereby causing a change in the hard tissue as the surgery simulation is performed.

In an embodiment, the change in the hard tissue may be achieved through a plurality of surgical operation. That is, the server 100 may perform the plurality of surgical operations for changing the hard tissue in the matching data 30.

The user interface 500 may be characterized in that a surgery simulation related to a plurality of surgical operations based on a setting value input from a user is provided. In an embodiment, the surgery simulation based on the setting value input from the user may be understood to mean that the plurality of surgical operations are performed on the basis of a numerical value input by the user through the hard tissue change information input screen 520.

In an embodiment, the plurality of surgical operations relate to double-jaw surgery and may include first to sixth surgical operations. The server 100 may provide the surgery simulation by subdividing it into several operations to be applicable to actual surgery. In an embodiment, the first to sixth surgical operations may be performed on the basis of one or more landmarks. The one or more landmarks relate to anatomical points on the head, and may include the orbitale, ANS, PNS, U1MP, U6MP, etc. but is not limited thereto. A plurality of surgical operations provided through a user interface will be described in detail with reference to FIGS. 6 to 8 below. In an embodiment, surgery related to the upper jaw will be described as an example in the following drawings and description, but it will be obvious to those of ordinary skill in the art that surgery related to the upper jaw is also applicable to various surgical sites, including the lower jaw and the mental region of the jaw, as well as the upper jaw.

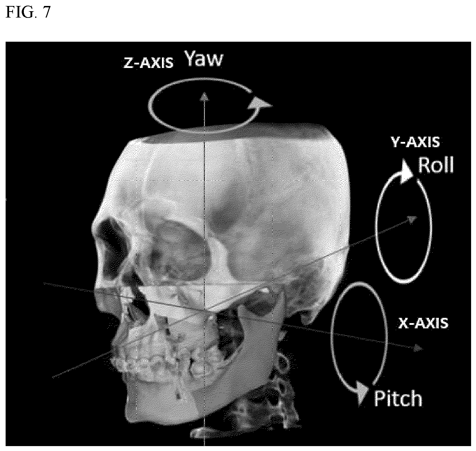

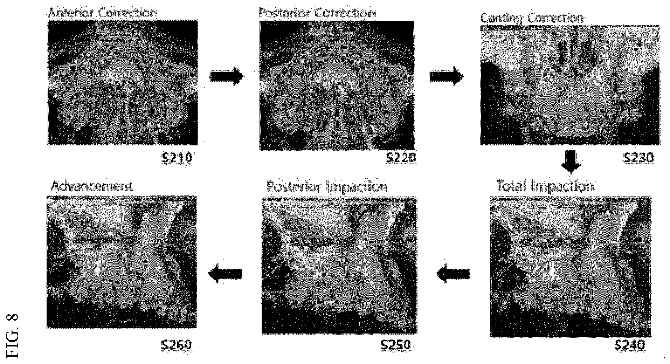

Specifically, the plurality of surgical operations may include a first surgical operation of performing movement on an x-axis. The first surgical operation may be related to anterior correction. As shown in FIGS. 7 and 8, the x-axis may be an axis related to left and right sides of the face when viewed from the front. In an embodiment, the hard tissue change information input screen 520 may include a first information input window for receiving a first input related to the first surgical operation from a user as shown in FIG. 6. The server 100 may perform the first surgical operation on the basis of the first input that is input from the user through the first information input window. For example, the first input may be information for moving the upper jaw to the left and right when the face is viewed from the front. That is, the server 100 may perform the first surgical operation related to the x-axis movement on the basis of the first input that is input to the first information input window of the hard tissue change information input screen 520.

The plurality of surgical operations may further include a second surgical operation of performing rotation on a z-axis with respect to an arbitrary reference point after applying the first surgery operation with respect to one or more landmarks. The second surgical operation may be related to posterior correction. As illustrated in FIGS. 7 and 8, the z-axis may be an axis related to the upper and lower sides of the face when viewed from the front, and the z-axis rotation may be yaw-related rotation. In an embodiment, the hard tissue change information input screen 520 may include a second information input window for receiving a second input related to the second surgical operation from the user as shown in FIG. 6. The server 100 may perform the second surgical operation on the basis of the second input that is input from the user through the second information input window. For example, the second input may be information for performing the z-axis rotation about maxillary central incisor (U1MP) until an x-axis coordinate of U6MP is changed by an input numerical value when the face is viewed from the front. That is, the server 100 may perform the second surgical operation related to the z-axis rotation about U1MP on the basis of the second input that is input to the second information input window of the hard tissue change information input screen 520.

The plurality of surgical operations may include a third surgical operation of performing rotation on a y-axis with respect to the arbitrary reference point after applying the second surgery operation with respect to one or more landmarks. The third surgical operation may be related to canting correction. As illustrated in FIGS. 7 and 8, the y-axis may be an axis related to the front and rear sides of the face when viewed from the front, and the y-axis rotation may be roll-related rotation. In an embodiment, the hard tissue change information input screen 520 may include a third information input window for receiving a third input related to the third surgical operation from the user as shown in FIG. 6. The server 100 may perform the third surgical operation on the basis of the third input that is input from the user through the third information input window. For example, the third input may be information for performing the y-axis rotation about the maxillary central incisor (U1MP) until a z-axis coordinate of U6MP is changed by an input numerical value when the face is viewed from the front. That is, the server 100 may perform the third surgical operation related to the y-axis rotation about U1MP on the basis of the third input that is input to the third information input window of the hard tissue change information input screen 520.

The plurality of surgical operations may include a fourth surgical operation of performing the z-axis movement. The fourth operation may be related to total impaction. As shown in FIGS. 7 and 8, the z-axis may be an axis related to upper and lower sides (i.e., the top and bottom) of the face when viewed from the front.

In an embodiment, the hard tissue change information input screen 520 may include a fourth information input window for receiving a fourth input related to the fourth surgical operation from the user as shown in FIG. 6. The server 100 may perform the fourth surgical operation on the basis of the fourth input that is input from the user through the fourth information input window. For example, the fourth input may be information for moving the upper jaw upward and downward when the face is viewed from the front. That is, the server 100 may perform the fourth surgical operation related to the z-axis movement on the basis of the fourth input that is input to the fourth information input window of the hard tissue change information input screen 520.

The plurality of surgical operations may include a fifth surgical operation of performing the x-axis rotation with respect to the arbitrary reference point after applying the fourth surgery operation with respect to one or more landmarks. The fifth operation may be related to posterior impaction. As illustrated in FIGS. 7 and 8, the x-axis may be an axis related to the left and right when the face is viewed from the front, and the x-axis rotation may be pitch-related rotation. In an embodiment, the hard tissue change information input screen 520 may include a fifth information input window for receiving a fifth input related to the fifth surgical operation from the user as shown in FIG. 6. The server 100 may perform the fifth surgical operation on the basis of the fifth input that is input from the user through the fifth information input window. For example, the fifth input may be information for performing the x-axis rotation about the maxillary central incisor (U1MP) until a z-axis coordinate of U6MP is changed by an input numerical value when the face is viewed from the front. That is, the server 100 may perform the fifth surgical operation related to the x-axis rotation about U1MP on the basis of the fifth input that is input to the fifth information input window of the hard tissue change information input screen 520.

The plurality of surgical operations may include a sixth surgery operation related to the y-axis movement. The sixth surgical operation may be related to AP movement. As shown in FIGS. 7 and 8, the y-axis may be an axis related to front and rear sides of the face when viewed from the front. In an embodiment, the hard tissue change information input screen 520 may include a sixth information input window for receiving a sixth input related to the sixth surgical operation from the user as shown in FIG. 6. The server 100 may perform the sixth surgical operation on the basis of the sixth input that is input from the user through the sixth information input window. For example, the sixth input may be information for moving the upper jaw forward and backward when the face is viewed from the front. That is, the server 100 may perform the sixth surgical operation related to the x-axis movement on the basis of the sixth input that is input to the sixth information input window of the hard tissue change information input screen 520.

As described above, the server 100 may receive numerical information related to the first to sixth surgical operations through a plurality of information input windows included in the hard tissue change information input screen 520 of the user interface 500, and perform a surgery simulation. The surgery simulation may be performed based on one or more landmarks related to anatomical points on the head (e.g., the orbitale, ANS, PNS, U1MP, U6MP, etc.), and performed through subdivided operations. As the surgery simulation is performed, a change in the hard tissue may be caused.

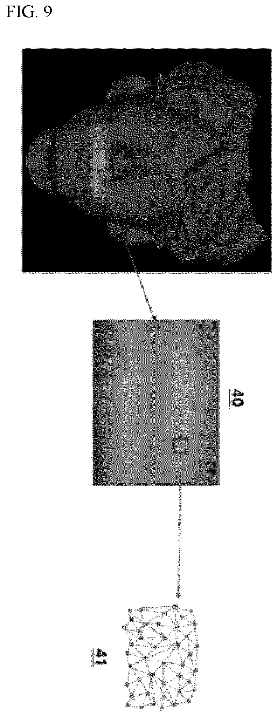

According to an embodiment, when the hard tissue changes, the server 100 may change soft tissue corresponding to the hard tissue using a weighted array. In an embodiment, the weighted array 40 is formed in relation to a surgical site, and a weighted array may be formed, for example, in relation to the upper jaw for maxillary surgery as shown in FIG. 9. The weighted array 40 may calculate a movement coefficient of soft tissue according to a change in hard tissue. In other words, when a change in the hard tissue occurs, the server 100 may change the soft tissue corresponding to change in the hard tissue.

In an embodiment, the weighted array may include a mesh 41 consisting of a plurality of vertices and edges connecting the plurality of vertices. Each of the plurality of vertices is matched to one of setting values according to a positional relationship with one or more landmarks. In other words, setting values about degrees to which the vertices are to be moved or forces to be applied to the vertices based on one or more landmarks may be matched to the vertices. The setting values may be calculated according to a clinical empirical rule and assigned to the vertices. The setting values corresponding to the vertices may be optimal values obtained as a result of clinical research related to various surgical situations. For example, a higher setting value may be matched to a vertex more adjacent to a first landmark related to an area with a high distribution of the skin (e.g., an area near the cheeks), and a lower setting value may be matched to a vertex more adjacent to a second landmark related to an area with a low distribution of the skin (e.g., the nose, the chin, or the like). The detailed description of the matching of the setting value is only an example and thus the present disclosure is not limited thereto. Each of the plurality of vertices included in the weighted array 40 formed in relation to the surgical site may be matched to individual setting values according to a positional relationship with one or more landmarks. Accordingly, influences on the vertices may be different from each other even when the same force is given (e.g., even when the amount of movement of the hard tissue is the same). That is, soft tissue is not moved by the same amount of movement of the hard tissue but vertices thereof may be moved differently on the basis of setting values assigned thereto according to a positional relationship with landmarks. Therefore, the soft tissue may be changed naturally as the hard tissue changes.

In an embodiment, when a force is applied to the plurality of vertices according to the movement of the hard tissue, the weighted array 40 may move the plurality of vertices on the basis of the applied force and the setting values matched to the plurality of vertices. The application of the force here may be related to the change in the hard tissue. For example, it may be understood that the applied force is large when a degree of change in the hard tissue is large and is small when the degree of change in the hard tissue is small. That is, when a force is applied to the vertices according to the change in the hard tissue, the movement of each of the vertices may be determined on the basis of the force applied to each of the vertices and the setting value matched to each of the vertices. For example, an amount of movement of a first vertex may be determined by a product (i.e., force*fn) of a force generated at the first vertex and a setting value fn matched to the first vertex.

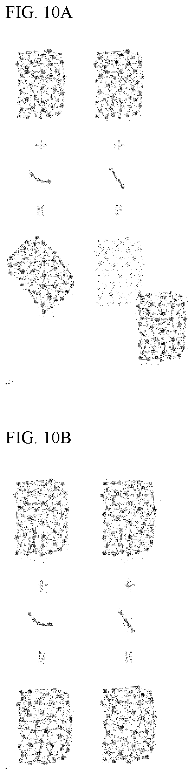

According to an embodiment, when a force in a specific direction (e.g., a vector) is applied to soft tissue of the skin, the soft tissue may be moved in response to the force as shown in FIG. 10A. However, because the skin corresponding to the soft tissue cannot move by itself, the weighted array 40 may be used to represent a natural change in the soft tissue according to the present disclosure. That is, when the weighted array 40 including the mesh 41 is used, only vertices to which a force is applied may be gradually moved to reproduce a change in the soft tissue as shown in FIG. 10B.

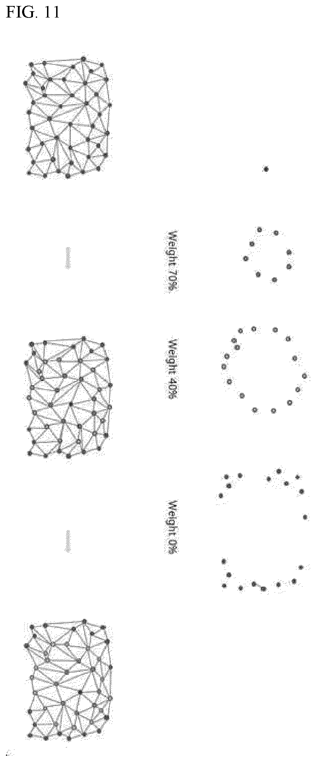

In an embodiment, different weights may be assigned to the plurality of vertices according to a depth of connection between adjacent vertices. Here, the weights may be understood as weights generated by a force. For example, referring to FIG. 11, a weight assigned to a part (e.g., a red vertex) at which a force is directly generated may be 100%, and small weights that decrease gradually (70%, 40%, and 0%) may be assigned according to depths of connection between vertices. According to an embodiment, an amount of movement of each vertex may vary according to a weight assigned to the vertex. For example, the amount of movement of a vertex assigned a weight of 100% may be large as shown in FIG. 11, and the amounts of movement of neighboring vertices may decrease gradually because weights assigned thereto decrease gradually according to a depth of connection with respect to the vertex. That is, the server 100 may use the weighted array 40 including the mesh 41 to simulate a gradual change in soft tissue according to a change in hard tissue.

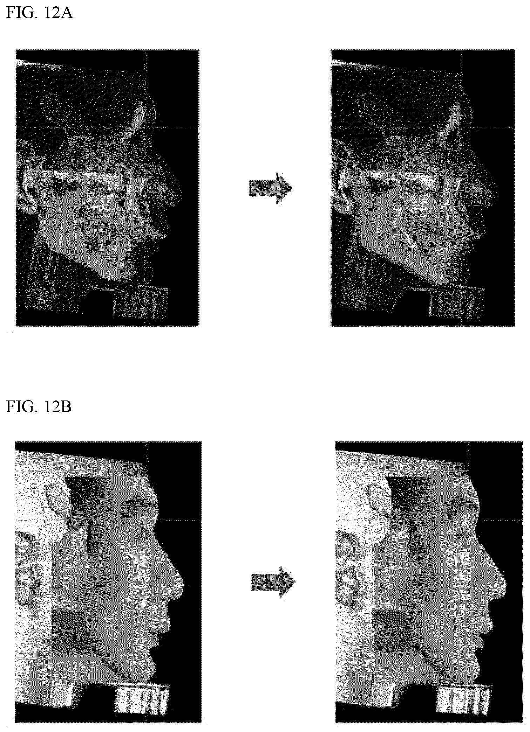

As described above, the server 100 of the present disclosure may perform a virtual surgery simulation (e.g., a surgery simulation) to change hard tissue with respect to one or more landmarks in matching data corresponding to a person who will undergo surgery instead of directly correcting soft tissue, and perform a simulation (i.e., a face transformation simulation) to change the soft tissue according to the change in the hard tissue as a result of the virtual surgery simulation. As a concrete example, when a change in hard tissue is caused by a surgery simulation as described in FIG. 12A, a change in soft tissue corresponding to the change in the hard tissue may be achieved using a weighted array as shown in FIG. 12B.

That is, as shown in FIG. 9, the server 100 may generate the weighted array 40 in relation to a surgical site on the basis of one or more landmarks, and provide a simulation for changing soft tissue (e.g., the skin) using the weighted array 40 according to movement of hard tissue. Accordingly, a user (e.g., a person who will perform a surgery) may obtain information about a change in soft tissue only on the basis of a change in hard tissue reflected as a result of performing a simulation related to the surgery without direct correction of the soft tissue, thereby improving convenience.

According to various embodiments of the present disclosure, it is possible to simulate a change in soft tissue corresponding to a change in hard tissue according to a procedure or surgery and provide a result of the simulation.

Effects of the present disclosure are not limited to the above effect, and other effects that are not described above will be clearly understood by those of ordinary skill in the art from the above detailed description.

The operations of the method or an algorithm described above in connection with embodiments of the present disclosure may be implemented directly by hardware, a software module executed by hardware, or a combination thereof. The software module may reside in a RAM, a ROM, an EPROM, an EEPROM, a flash memory, a hard disk, a removable disk, a CD-ROM, or any type of computer-readable recording medium well-known in the technical field to which the present disclosure pertains.

Components of the present disclosure may be embodied in the form of a program (or an application) and stored in a medium to be executed in combination with a computer which is hardware. The components of the present disclosure may be implemented by software programming or software elements, and similarly, embodiments may be implemented in a programming or scripting language such as C, C ++, Java, or an assembler, including data structures, processes, routines, or various algorithms which are combinations of different programming constructs. Functional aspects may be embodied as an algorithm executable by one or more processors.

It will be understood by those of ordinary skill in the art that various types of logic blocks, modules, processors, means, circuits, and operations of algorithms described above as examples in relation to the embodiments set forth herein are implementable using electronic hardware, various types of programs or design code (referred to as "software" herein for convenience of description), or a combination thereof. To clearly describe the interoperability between hardware and software, various types of components, blocks, modules, circuits, and operations have been generally described above as examples in relation to functions thereof. Whether such a function is implemented as hardware or software depends on a specific application and design restrictions imposed on the entire system. Those of ordinary skill in the art can implement functionalities in various ways for each specific application, but decisions of such implementation should not be understood as departing from the scope of the present disclosure.

The various embodiments set forth herein may be implemented as articles manufactured by methods, apparatuses, or standard programming and/or engineering techniques. The term "manufactured article" should be understood to include a computer program, a carrier or media accessible by any computer-readable device. Examples of a computer-readable medium may include, but are not limited to, magnetic storage devices (e.g., a hard disk, a floppy disk, a magnetic strip, etc.), optical disks (e.g., a CD, a DVD, etc.), smart cards, and flash memory devices (e.g., an EEPROM, a card, a stick, a key drive, etc.). In addition, the various types of storage media presented herein include one or more devices for storing information and/or other machine-readable media. The term "machine-readable media" includes, but is not limited to, wireless channels and various other media for storing, retaining, and/or transmitting instruction(s) and/or data.

It should be understood that the specific order or hierarchy of operations of each of the presented processes is an example of exemplary approaches. It should be understood that a specific order of hierarchical structure of operations of a process within the scope of the present disclosure may be rearranged on the basis of design priorities. The appended method claims provide elements of various operations in a sample order but should not be understood as being limited to the specific order or hierarchical structure presented herein.

A description of embodiments set forth herein is provided to help those of ordinary skill in the art use or implement the present disclosure. It will be obvious to those of ordinary skill in the technical field of the present disclosure that various modifications may be made in these embodiments, and the general principles defined herein may be applied to other embodiments without departing from the scope of the present invention as defined by the claims.

## Claims

1. A method of performing a face transformation simulation, which is performed by one or more processors of a computing device, the method comprising:
obtaining three-dimensional (3D) volume data and 3D facial data;
generating matching data by matching the 3D volume data and the 3D facial data;
generating a user interface including the matching data and providing the user interface to a user terminal; and
performing transformation on the matching data on the basis of a response to the user interface, the response being received from the user terminal.

2. The method of claim 1, wherein the 3D volume data is volume data related to the head and neck of a person who undergoes surgery and comprises one or more landmarks, and
the 3D facial data is image data related to the face of the person who undergoes the surgery and comprises polygon data and texture data.

3. The method of claim 1 or 2, wherein the generating of the matching data comprises:
identifying landmarks for matching in the 3D volume data and the 3D facial data; and
generating the matching data on the basis of the identified landmarks for matching.

4. The method of any one of the preceding claims, wherein the user interface comprises:
a simulation selection screen for receiving a selection input related to an orthodontic simulation or a plastic and orthognathic surgery simulation from a user;
a matching data display screen displaying the matching data; and
a hard tissue change information input screen for receiving a hard tissue adjustment input in relation to a change in hard tissue from the user.

5. The method of claim 4, wherein the performing of the transformation on the matching data comprises:
changing the hard tissue on the basis of the hard tissue adjustment input; and
changing soft tissue corresponding to the hard tissue using a weighted array when the hard tissue is changed,
wherein the weighted array calculates a movement coefficient of the soft tissue according to the change in the hard tissue.

6. The method of claim 5, wherein the changing of the hard tissue is performed through a plurality of surgical operations,
wherein the plurality of surgical operations comprise:
a first surgical operation of performing an x-axis movement;
a second surgical operation of performing a z-axis rotation with respect to an arbitrary reference point after applying the first surgery operation with respect to one or more landmarks;
a third surgical operation of performing a y-axis rotation with respect to the arbitrary reference point after applying the second surgery operation with respect to the one or more landmarks;
a fourth surgical operation of performing the z-axis movement;
a fifth surgical operation of performing the x-axis rotation with respect to the arbitrary reference point after applying the fourth surgery operation with respect to the one or more landmarks; and
a sixth surgical operation of performing a y-axis movement.

7. The method of claim 5 or 6, wherein the weighted array comprises a mesh including a plurality of vertices and a plurality of edges connecting the plurality of vertices,
wherein the plurality of vertices are matched to setting values according to a positional relationship with one or more landmarks.

8. The method of claim 7, wherein, when a force is applied to the plurality of vertices according to movement of the hard tissue, the weighted array moves the plurality of vertices on the basis of the applied force and the setting values matched to the plurality of vertices, and
the plurality of vertices are assigned different weights according to depths of connection between adjacent vertices.

9. An apparatus for performing a face transformation simulation, comprising:
a memory storing one or more instructions; and
a processor configured to execute the one or more instructions stored in the memory to perform the method of any one of the preceding claims.

10. A computer program which when executed by one or more processors is arranged to perform a method according to any one of claims 1 to 8.
